# EUROPEAN PATENT SPECIFICATION

(11) **EP 1 614 696 B1**
(45) Date of publication and mention of the grant of the patent: **12.09.2007**
(21) Application number: 05014528.3
(22) Date of filing: 05.07.2005
(51) Int. Cl.: C08B 11/20, C08B 11/15, C08B 37/00, C08B 31/08, C08B 31/12, C08B 37/04, A61L 27/20, A61K 47/36, A61K 47/38

(54) **N-methyl-amide derivatives of Carboxymethylcellulose, alginic acid N-methyl-amide or carboxymethyl starch**
N-methyl-amide-Derivate von Carboxymethylcellulose, Alginsäure oder Carboxymethylstärke
Dérivés N-methyl-amide de la carboxyméthylcellulose, de l' acide alginique ou de l'amidon carboxyméthyle

(30) Priority: 09.07.2004 IT MI20041373
(43) Date of publication of application: 11.01.2006
(73) Proprietor: LIMA Lto SpA, 33030 Villanova di San Daniele del Friuli (IT)
(72) Inventor: Facchini, Alessandro, 33170 Pordenone (IT); Segatti, Francesco, 33100 Udine (IT)
(74) Representative: Banfi, Paolo

(56) References cited:
- EP-A- 0 342 557
- WO-A-00/27886
- US-A- 2 881 161
- US-A- 5 856 299
- DATABASE WPI Section Ch, Week 199014 Derwent Publications Ltd., London, GB; Class A97, AN 1990-106216 XP002349957 & SU 1 509 356 A (LAPENKO V L) 23 September 1989 (1989-09-23)

## Description

The present invention relates to carboxymethylcellulose, alginic acid or carboxymethyl starch N-methyl-amides and the use thereof in the biomedical field.

The use of natural macromolecules in the alimentary, medical/pharmaceutical, dermo-cosmetic and personal care fields is well established, due to their chemical-physical, technological and biological properties, as well as the low costs of the starting materials. Natural macromolecules are used in the alimentary field as thickening agents, viscosity-increasing agents, buffers, and the like, in the pharmaceutical field as lubricants, retardants, coatings and the like, for use in the production of both conventional (tablets, capsules, colloids, topical gels, etc.) and drug controlled-release formulations. In the medical field, natural biocompatible macromolecules are used to manufacture a number of medical devices (MD) (such as gauzes, powders, odor absorbers and liquids, occlusive pastes, injectable cutaneous fillers) and to coat articles (such as vascular catheters or cardiac valves), made from materials having mechanically suitability but reduced blood or tissue biocompatibility. A number of properties of cosmetic formulations (such as the hydrating property) are defined and ascribed based on the presence of suitable macromolecules in the compositions.

Exhaustive reviews on the characteristics and various uses of macromolecules are reported in the following publications and bibliographic references: "Handbook of Pharmaceutical Excipients" - A. Wade and P.J. Wellers, Editors - The Pharmaceutical Press, 1994; "Integrated Biomaterial Science" - R. Barbucci Editor - Kluwer Academic/Plenum Publ., 2002; "Handbook of Cosmetic Science and Technology" - A.OR. Barel et al. Editors-Marcel Dekker Inc., 2001.

The most used natural macromolecules of extractive origin are: celluloses (CEL), alginic acid (AA), starches (AM), pectins, various glycosaminoglycans or GAGs (heparins; chondroitin sulfates; heparin sulfates; hyaluronic acids, HAs, etc.); the most known and used semi-synthetic macromolecules (resulting from minor changes in the natural ones) are: carboxyalkyl-cellulose (carboxymethyl-cellulose, CMC); carboxyalkyl-starches (carboxymethyl-starch, CMA); carboxyalkyl-dextrans; carboxyalkyl-glucans, and the like.

The technological and biological characteristics of the various derivatives can remarkably differ, but their industrial use mainly depends on their costs.

For example, amorphous CEL is a polysaccharide with good liquid- and essudates-absorbing ability, which can be worked in threads, tissues, etc. and is available at low costs; all said factors make it ideal for the preparation of low cost MD, such as gauzes, absorbent pads, etc., finished products. Conversely, crystalline CEL, which is more expensive than the amorphous species due to the high purity required, shows fairly good swelling properties in the presence of liquids and is an excellent disintegrant agent for oral pharmaceutical formulations, which can have higher price.

AAs in the presence of divalent inorganic cations give raise to very hydrated physical hydrogels and are used as thickening agents in the food industry, due to their low cost. CMC and CMA have similar behaviour.

From the biological point of view, CEL, AA, CMC or CMA are more or less slowly degraded by bacteria and enzymatic systems, such as lyases, or by macrophages; for this reason and their low costs, they are used in those medical preparations in which no blood absorption is involved and in cosmetics.

Heparin is a highly expensive GAG with antithrombotic activity used, inter alia, to coat the surfaces of vascular catheters made from materials with suitable mechanic characteristics but low blood biocompatibility and therefore liable to induce thrombi. The importance of the final application and the high cost of the starting material made heparin poorly attractive form the industrial point of view.

To date, hyaluronic acids (HA) proved to be the most valuable and versatile biomaterials. High molecular HAs form water-rich gels, with variable viscosity but lacking viscoelasticity; they have better biocompatibility towards tissues and blood than CMC and AA, and are free from cytotoxicity; they are degraded (in some cases even too quickly) by specific enzymatic systems (hyaluronidase) which produce re-absorbable, harmless metabolites; upon suitable chemical modifications they can be worked in films and threads free from intrinsic mechanical resistance; they can be sterilized (although with due caution to avoid degradation processes which would affect their performances) and are injectable. The different HA gels, particularly upon chemical modification, are used, in the form of spreadable formulations, films, injectable formulations, etc., - for both class III MD (such as anti-tissue adhesion agents in abdominal surgery, cutaneous fillers in dermocosmesis; viscous injectable agents in osteoarthritis, wound healing agents for chronic ulcers, etc.) and for high performance moisturizers in cosmetics.

The industrial use of HA gels is restricted by:
- reduced *in vivo* half-life;
- absence of marked viscoelasticity characteristics;
   which in turn restrict or make their uses less valid, namely visco-supplementation in osteoarthritic joints and cutaneous filling action in dermatology: in both cases the effectiveness markedly decreases in time, which affects costs of therapeutical cycles;
- the high cost of the starting material, which further affects the above mentioned aspects.

HA is obtained by extraction from animal tissues and/or by fermentation from genetically modified microrganisms. In recent years, different innovations potentially able to optimize HA production processes have been described, which however did not significantly changed the final costs of the starting material (particularly in the case of high molecular HAs with constant, reproducible composition).

At the same time, a number of structural changes of HA have been suggested for different purposes, among them:
a) esterification of the carboxylic groups with alcohols;
b) amidation of the acid groups. WO 0001733 claims amides obtained from benzyl-, octyl-, dodecyl- and hexadecyl amines and HA or HA partially esterified with various biologically active alcohols. WO 8902445 discloses HA amides with amino acids esters and aniline. US 5,856,299 claims highly reactive esters of carboxy-polysaccharides, among them: HA, alginic acid, carboxymethylcellulose, etc.; said activated esters are potentially useful for the preparation of amides with primary amines (for example ethylamine, propylamine, isopropylamine, butylamine, benzylamine, etc.); the synthesis of these products is exemplified, but their characteristics are non described. According to the inventors, the changes should:
   1. increase the half-life of the formulation products through reduction of the enzymatic or bacterial degradation rate. This statement can be understood from the technical point of view when the change consists in the introduction in the macromolecule of highly hindered and lipophilic groups (phenyl, benzyl, n-propyl, iso-propyl octyl, hexadecyl, neutral amino acyl residues etc.), able to sterically affect the conformation of the polysaccharide, i.e. highly ionic residues capable of modifying the substrate conformation through highly stable hydrogen bonds (as it happens when sulfate groups are introduced in HA: *inter alia* A. Suzuki et Al., Glycobiology, 11, 57-64, 2001; T. Toida et Al., Intern. J. Biol Macromol., 26, 233-241, 1999).
      From what stated above, however, the behaviour of HA ethylamide is neither described nor expected or anticipated;
   2. modulate such characteristics as viscosity, water solubility, swelling, depending on the availability of free carboxy groups.

However, such modifications do not improve or solve problems concerning the costs of the starting material and the lack of viscoelasticity of HA gels.

Cross-linking of HA with suitable difunctional agents affords viscoelasticity, good enzymatic resistance (although to the detriment of solubility and swellability) and formation of hydrogels. Examples of cross-linking with various agents are reported in US 4,716,224; US 4,957,744; US 4,582,865; US 4,605,601; US 4,713,448; US 5,128,326; US 5,356,883; EP-A-718312; EP 1,144,459.

Considering the prior art, there is therefore a remarkable industrial need for products characterized by:
1) modulable, reproducible behaviour in terms of: viscosity, swelling, viscoelasticity and tixotropy of aqueous dispersions/solutions, comparable to that of HA or amido, ester or cross-linked derivatives thereof;
2) workability in various forms, such as: threads, tissues, foils, spreadable or injectable gels;
3) easy sterilizability of bulk, semi-manufactured or finished products;
4) similar cytotoxicity to that of HA, or the above derivatives thereof, and better than that of CMC, AA or CMA;
5) low cost of starting materials and derivatives thereof (included the cross-linked ones), thus making the products suitable for use in the alimentary, pharmaceutical, medical and cosmetic fields, even for low cost applications.

It has now surprisingly been found that carboxymethylcellulose, alginic acid and carboxymethyl starch N-methyl-amides have said characteristics.

Although hyaluronic acid amides (obtained from straight aliphatic amines, aromatic amines or amino acids esters) are known and amides of other carboxylated polysaccharides have been suggested but not described, HA, CMC, AA and CMA N-methyl-amides have not been described to date. Moreover, the chemical-physical characteristics - such as their water solubility - and the rheological characteristics of their aqueous solutions - such as viscosity, swelling, etc. - could not be expected or predicted on the basis of the characteristics of the said HA amides and of the starting CMC, AA or CMA.

An object of the present invention are carboxymethylcellulose, alginic acid and carboxymethyl starch N-methyl-amides in which the N-methyl amidation percentage of the starting carboxylic groups in the polysaccharide (AD) is lower than 100%. Said products have unexpected chemical-physical, rheological and biological characteristics which fulfill the above mentioned requirements and are much cheaper than HA, as they derive from inexpensive starting materials and less costly production processes.

Starting materials for the preparation of the amides of the invention can be selected from AA, CMC and CMA. The latter can have methylcarboxylation degree (MCD: number of carboxyls per repeating monosaccharide units) ranging from 0.1 to 1, preferably from 0.5 to 1.

The N-methyl-amides of the invention can be prepared from methyl-amine base, or salts thereof, and the tetrabutylammonium (TBA) salt of the suitable carboxylated polysaccharide, which is in turn prepared starting from the sodium salt or the polymer acidic form. Condensation between methyl-amine and TBA salt takes place upon activation of the carboxylic groups by known reagents, optionally in the presence of suitable catalysts.

The reaction conditions can vary depending on the activator used. For example, when activation is carried out with chloromethylpyridinium iodide (CMPJ) - as disclosed, inter alia, in EP 1,144,459 or dicyclohexylcarbodiimide (DCC) - anhydrous aprotic solvents (such as dimethylformamide, DMF, or dimethylsulfoxide, DMSO) are used.

On the other hand, when activation is carried out with agents such as water soluble carbodiimides (WSC), an aqueous reaction medium can be used.

The substitution or amidation degree (AD) - which is the percentage of carboxylic groups in the starting polysaccharide which are transformed into methylamido groups - can range from 1% to 100% on the theoretical, depending on the desired characteristics of the starting polycarboxyamido polymer or derivatives thereof, such as cross-linked hydrogels.

Depending on the selected process, the factors affecting (in a reproducible manner) the AD can be either the amount of methylamine added after activation (use of DCC) or the amount of CMPJ used, as described in the examples below. The formation of the amido groups and the evaluation of AD can be carried out with conventional analytic techniques, such as IR spectroscopy (appearance of an absorption band at 1624 cm⁻¹, stretching of C=OR amido) and nuclear magnetic resonance. The resulting CMC, AA and CMA methyl-amides are water-soluble (even at AD = 100%) and have viscosities depending on the CMD ratio of the starting polysaccharide and the selected AD.

A further object of the invention are the cross-linked derivatives obtained from the above methylamides (with AD < 100%) by procedures similar to those used in primary amidation, for example via activation of the residual free carboxylic groups and reaction with di-amines (for example: propanediamine, hexanediamine etc.) or di-alcohols (1.2 ethylene glycol, PEG) esters of basic amino acids such as lysine, and formation of hydrogels.

Said cross-linked derivatives can have different cross-linking degrees (CLD), expressed as the percentage of the free carboxylic groups remaining after amidation, involved in the cross-linking reaction. By way of examples, these derivatives can have 50% AD and 50% CLD and 25% acid groups of the starting polysaccharide, i.e. 50% AD and 100% CLD, and contain no acidic functions. The resulting hydrogels have different chemical-physical characteristics; for example, CMC-A hydrogels (amido- CMC) with 50% AD and 50% CLD have higher swellabilty than those with 50% AD and 100% CLD, which conversely have higher viscoelasticity. The first are still able to interact with inorganic cations - such as calcium or iron ions - or organic bases (biologically active or inert), contrary to those with 50% AD and 100% CLD. Peculiarly, CMC-A and propanediamine hydrogels with 50% AD and 100% CLD have physical-rheological characteristics (such as water-uptake or viscosity) very close to those of the corresponding hydrogels from HA, with 100% CLD and with the same cross-linking agent. In principle, both CMC methyl-amides and the corresponding hydrogels have lower cytotoxicity than the starting polysaccharides.

A further object of the present application is the treatment of hydrogels from AA with AD ranging from 10% to 50% and 50% CLD, independently of the nature of the cross-linking agent - with inorganic cations such as calcium, iron etc.. Said products have peculiar ability as thickening agents. Due to their chemical-physical and rheological characteristic and also to their low cytotoxicity, the products of the present invention are suitable for a number of applications, such as the production of: intestinal drug delivery systems of pharmacologically active principles; mucus and dermal adhesive moisturizing formulations; medical devices (MD) for the therapy of degenerative osteo-arthritis; MD for healing chronic ulcers or bums; preparation of highly biotolerated urethral catheters; highly moisturizing dermocosmetic formulations.

The following examples illustrate the invention in greater detail.

Both AD and CLD are determined by titration of the free carboxyls with 0.1N NaOH.

### Example 1: Synthesis of carboxymethylcellulose methylamide (CMC-A) with 50% AD by activation of CMC carboxyls with chloromethylpyridinium iodide (CMPJ)

CMC tetrabutylammonium (TBA) salt with CMD 0.8 (PM 920) is prepared by percolating a solution of CMC sodium salt on a DOWEX Monosphere 650 C column in acid form. Acid CMC eluate is then adjusted to pH 8-9 with a TBA base 0.5M solution. The solution is then filtered and freeze-dried.

3 g of TBA salt (3/920 = 3.2 x 10⁻³ mols of repeating units corresponding to 50% -COOH groups) are dissolved in 300 ml of dry DMF under nitrogen; the solution is cooled to 4°C and added with 0.83 g of chloromethylpyridinium iodide (CMPJ), corresponding to the activation of 50% of the CMC carboxylic groups. After stirring al 4°C for 4-6 hours, 0.10 g of freshly distilled methylamine (density: 0897), equivalent to 0.11 ml and ½ drop of triethylamine are slowly added, as catalyst. The solution is left under stirring while temperature spontaneously returns to the room one. The amidated polymer (CMC-A) is precipitated with an excess of dry ethyl ether centrifuged and redissolved in the minimum amount of DMF. DMD and unreacted methylamine are evaporated off under vacuum and the product is recovered.

### Characterization of the derivative:

IR: band at 1624 cm⁻¹ (amido C=O stretching); shift of the band at 1597 cm⁻¹ (COO- stretching) at 1.580 cm⁻¹ by contribution of the amido NH bending.

### Example 2: Synthesis of carboxymethylcellulose methylamide (CMC-A) with 100% AD by activation of CMC carboxyls with chloromethylpyridinium iodide (CMPJ)

The product is prepared according to Example 1 using 3 g of CMC TBA salt and 0.170 g of CMPJ. Final product are recovered as described above.

### Characterization of the product:

IR: band at 1624 cm⁻¹ (amido C=O stretching)

The characteristics correspond to a CMC in which all the carboxylic groups are amidated.

### Example 3: Synthesis of carboxymethylcellulose methylamide (CMC-A) with 50% AD by activation of CMC carboxyls with dicyclohexylcarbodiimide (DCC)

3 g of CMC TBA salt, prepared according to Example 1, are dissolved in approx. 300 ml of dry DMF and under nitrogen. 2.69 g of DCC dissolved in 10 ml of dry DMF and 0.11 ml (0.11 g) of methylamine are added, under stirring. Stirring is continued for 4 hours and the polymer is recovered as described in Example 1. The chemical-physical characteristics are the same as those of the product described in Example 2.

### Example 4: Synthesis of carboxymethylcellulose methylamide (CMC-A) with 100% AD by activation of CMC carboxyls with dicyclohexylcarbodiimide (DCC)

The product is prepared as described in Example 3, using 3 g of CMC, 5.38 g of DCC and 0.22 g of methylamine. Recovery is carried out as in Example 1. The chemical-physical characteristics are the same as those of the product described in Example 2.

### Example 5: Synthesis of carboxymethylcellulose methylamide (CMC-A) with 5% AD by activation of CMC carboxyls with EDC (water soluble carbodiimide)

3 g of CMC sodium salt are dissolved in 300 ml of distilled water and added with 4.77 g of EDC (molar ratio CMCNa: EDC 1:4). After that, 0.214 ml of methylamine are slowly added, keeping the pH at 4.6-4.9 by addition of highly dil. HCl. The product is recovered by threefold dilution of the final volume with absolute ethanol. The dry product has the same characteristics of 50% AD CMC-A as the above examples.

### Example 6: Synthesis of 100% CLD cross-linked CMC-A from CMC-A 50% AD and diamino-propane in the presence of CMPJ

3 g of CMC-A TBA salt prepared as described in Example 1 are dissolved in 300 ml of dry DMF, under nitrogen and with stirring. The solution is adjusted to 4°C and added with 0.83 g of CMPJ dissolved in 2 ml of DMF. After stirring for 30 min, 0.120 g of diaminopropane (density 0.88), equivalent to 0.13ml, and 4/5 drops of triethylamine are added. The solution is reacted for 3 h, then diluted with water and the precipitated gel is recovered, repeatedly washed with ethanol and water and dried under vacuum at r.t. or by freeze-drying.

### Characterization:

### - Hydration behaviour (swelling):

### Control products of:

1) CMC hydrogel (100% CMC) obtained by cross-linking with 100% CLD diaminopropane, according to WO-A-0027887;
2) HA hydrogel (100% Hyal) obtained by cross-linking with 100% CLD diaminopropane, according to WO-A-0027887.

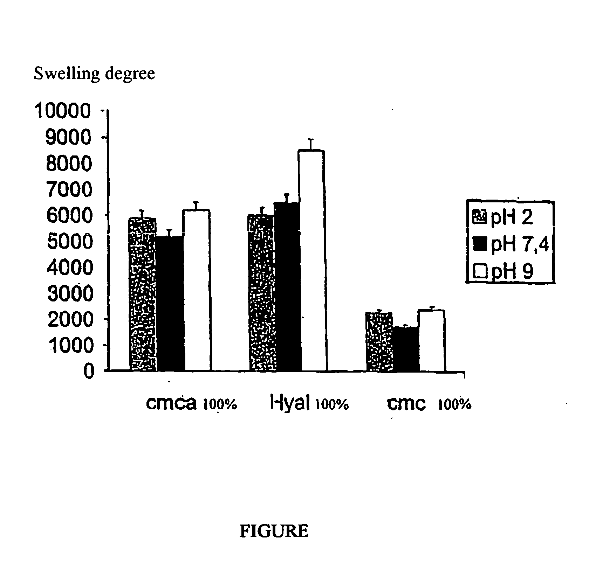

Swelling tests were carried out at three pH values: 2; 7,4; 9. Results are reported in Figure 1. It is evident that at acid or neutral pH, CMC-A hydrogel with 50% AD and with 100% CLD behave similarly to the HA hydrogel of point 2, both being markedly superior to that of 100% CMC (point 1).

### Example 7: Evaluation of cytotoxicity on osteoblasts

The following products were subjected to the cytotoxicity test:
- CMC cross-linked with 50% CLD propanediamine, prepared according to WO-A-0027887;
- 50% AD CMC-A, and 100% CLD of Example 6;
- hyaluronic acid cross-linked with 50% CLD propanediamine, prepared according to WO-A-0027887.

Cells used: osteoblasts MG63 were monitored for the following parameters: cell proliferation by colorimetric method (WST1: measurement of cell viability based on mitochondrial metabolic activity); production in the supernatant of: lactic dehydrogenase (LDH), interleukin 6 (IL6) and Tumor Necrosis Factor (TNF-(α) as indications of cell suffering and inflammatory reaction.

Results are reported in the following table.

**Table**

| MG63 | Control CMC hydrogel of WO-A-0027887 | CMC50% | CMC-A | HYAL50% |
|---|---|---|---|---|
| WST1 | 0.67 ± 0.4 | 0.63 ± 0.26 | 0.81 ± 0.14 | 0.83 ± 0.10 |
| LDH | 12.4 ± 1.9 | 18.0 ± 3.9 | 15.8 ± 5.2 | 21.4 ± 1.9 |
| IL-6 | 0.7 ± 0.06 | 8.23 ± 1.74 | 1.87 ± 0.27 | 3.2 ± 1.00 |
| TNF-alpha | 8.15 ± 0.72 | 7.08 ± 1.1 | 6.87 ± 1.03 | 8.32 ± 1.42 |

## Claims

1. Carboxymethylcellulose, alginic acid or carboxymethyl starch N-methyl-amides in which the N-methyl-amidation percentage of the starting carboxylic groups in the polysaccharide (AD) is lower than 100%.

2. Carboxymethylcellulose N-methyl-amides according to claim 1.

3. Alginic acid N-methyl-amides according to claim 1.

4. Carboxymethyl starch N-methyl-amides according to claim 1.

5. N-methyl-amides as claimed in any one of claims 2 or 4 in which the number of carboxymethyls per monosaccharide unit (CMD) ranges from 0.1 to 1.

6. N-methyl-amides as claimed in claim 5, in which the number of carboxymethyls per monosaccharide unit (CMD) ranges from 0.5 to 1.

7. N-methyl-amides as claimed in any one of claims 1 to 6 with AD lower than 100%, cross-linked by reaction between the free carboxylic groups, suitably activated, and diamines, diols or esters of basic amino acids.

8. N-methyl-amides as claimed in claim 7 in which part of the carboxylic groups are reacted with propanediamine, hexanediamine, 1,2-ethylene glycol, PEG, lysine esters alkyl esters.

9. N-methyl-amides as claimed in claims 7 or 8 in the form of hydrogels.

10. N-methyl-amides as claimed in claims 7 to 9 having cross-linking degree (CLD) ranging from 50% to 100% of residual non methylamidated carboxylic groups.

11. N-methyl-amides as claimed in claims 8 and 10 with 10% to 50% AD and 50% CLD in the form of salts with inorganic cations.

12. The use of carboxymethylcellulose, alginic acid or carboxymethyl starch N-methyl-amides of claims 1 to 11 as drug delivery systems of pharmacologically active principles; mucus and dermal adhesive moisturizing formulations; medical devices for the therapy of degenerative osteo-arthritis; medical devices for healing chronic ulcers or burns; preparation of highly biotolerated urethral catheters; highly moisturizing dermocosmetic formulations; cell scaffolds.

## Patentansprüche

1. Carboxymethylcellulose-, Alginsäure- oder Carboxymethylstärke-N-Methylamide, bei denen der Prozentsatz der N-Methylamidierung der Ausgangs-Carboxylgruppen im Polysaccharid (AD) geringer als 100% ist.

2. Carboxymethylcellulose-N-Methylamide gemäß Anspruch 1.

3. Alginsäure-N-Methylamide gemäß Anspruch 1.

4. Carboxymethylstärke-N-Methylamide gemäß Anspruch 1.

5. N-Methylamide wie in einem der Ansprüche 2 oder 4 beansprucht, in denen die Anzahl an Carboxymethylgruppen pro Monosaccharideinheit (CMD) von 0,1 bis 1 reicht.

6. N-Methylamide wie in Anspruch 5 beansprucht, in denen die Anzahl an Carboxymethylgruppen pro Monosaccharideinheit (CMD) von 0,5 bis 1 reicht.

7. N-Methylamide wie in einem der Ansprüche 1 bis 6 beansprucht mit einem AD-Wert geringer als 100%, die durch Reaktion zwischen den freien, geeignet aktivierten Carboxylgruppen und Diaminen, Diolen oder Estern von basischen Aminosäuren vernetzt sind.

8. N-Methylamide wie in Anspruch 7 beansprucht, in denen ein Teil der Carboxylgruppen mit Propandiamin, Hexandiamin, 1,2-Ethylenglycol, PEG, Lysinesteralkylester reagiert ist.

9. N-Methylamide wie in Anspruch 7 oder 8 beansprucht in der Form von Hydrogelen.

10. N-Methylamide wie in den Ansprüchen 7 bis 9 beansprucht, die einen Vernetzungsgrad (CLD) von 50% bis 100% der verbliebenen nichtmethylamidierten Carboxylgruppen aufweisen.

11. N-Methylamide wie in den Ansprüchen 8 und 10 beansprucht mit einem AD-Wert von 10% bis 50% und einem CLD-Wert von 50% in der Form von Salzen mit anorganischen Kationen.

12. Verwendung von Carboxymethylcellulose-, Alginsäure- oder Carboxymethylstärke-N-Methylamiden der Ansprüche 1 bis 11 als Arzneimitteltransportsysteme von pharmakologisch aktiven Wirkstoffen; Schleim- und Haut-anhaftende feuchtigkeitsspendende Formulierungen; Medizinprodukte für die Therapie von degenerativer Osteoarthritis; Medizinprodukte für die Heilung von chronischen Geschwüren oder Verbrennungen; Herstellung von hochgradig biotolerierten Harnröhrenkathetern; hochgradig feuchtigkeitsspendende hautkosmetische Formulierungen; Zell-Scaffolds.

## Revendications

1. N-méthyl-amides de carboxyméthylcellulose, acide alginique ou carboxyméthyl amidon dans lesquels le pourcentage de N-méthyl-amidation des groupes carboxyliques de départ dans le polysaccharide (AD) est inférieur à 100 %.

2. N-méthyl-amides de carboxyméthylcellulose selon la revendication 1.

3. N-méthyl-amides d'acide alginique selon la revendication 1.

4. N-méthyl-amides de carboxyméthyl amidon selon la revendication 1.

5. N-méthyl-amides tels que revendiqués dans l'une quelconque des revendications 2 ou 4, dans lesquels le nombre de carboxyméthyles par unité de monosaccharide (CMD) s'étend de 0,1 à 1.

6. N-méthyl-amides tels que revendiqués dans la revendication 5, dans lesquels le nombre de carboxyméthyles par unité de monosaccharide (CMD) est dans l'intervalle de 0,5 à 1.

7. N-méthyl-amides tels que revendiqués dans l'une quelconque des revendications 1 à 6 avec un AD inférieur à 100 %, réticulés par réaction entre les groupes carboxyliques libres, activés de manière appropriée, et des diamines, diols ou esters d'acides aminés basiques.

8. N-méthyl-amides tels que revendiqués dans la revendication 7, dans lesquels une partie des groupes carboxyliques est mise en réaction avec propanediamine, hexadiamine, 1,2-éthylène glycol, PEG, esters d'alkyle d'esters de lysine.

9. N-méthyl-amides tels que revendiqués dans les revendications 7 ou 8, sous forme d'hydrogels.

10. N-méthyl-amides tels que revendiqués dans les revendications 7 à 9 ayant un degré de réticulation (CLD) s'étendant de 50 % à 100 % de groupes carboxyliques non méthylamidés résiduels.

11. N-méthyl-amides tels que revendiqués dans les revendications 8 et 10 avec un AD de 10 % à 50 % est un CLD de 50 %, sous forme de sels avec des cations inorganiques.

12. Utilisation de N-méthyl-amides de carboxyméthylcellulose, acide alginique ou carboxyméthyl amidon selon les revendications 1 à 11 comme systèmes d'administration de médicaments pour des principes actifs sur le plan pharmacologique ; formulations hydratantes adhésives du mucus et du derme ; dispositifs médicaux pour la thérapie de l'arthrose ; dispositifs médicaux pour soigner les ulcères chroniques ou les brûlures ; préparation de cathéters urétraux fortement biotolérés ; formulations dermocosmétiques fortement hydratantes ; support de cellules.
